# EUROPEAN PATENT SPECIFICATION

(11) **EP 0 336 487 B1**
(45) Date of publication and mention of the grant of the patent: **13.10.1993**
(21) Application number: 89200789.9
(22) Date of filing: 28.03.1989
(51) Int. Cl.: A61F 11/06

(54) **Hearing protector**
Gehörschützer
Protecteur auditif

(30) Priority: 28.03.1988 NL 8800780
(43) Date of publication of application: 11.10.1989
(73) Proprietor: Verenigde Bedrijven Groeneveld B.V., NL-3316 GB Dordrecht (NL)
(72) Inventor: van Tussenbroek,Johan Paul, 4461 KE Goes (NL)
(74) Representative: Boelsma, Gerben Harm, Ir.

(56) References cited:
- FR-A- 491 801
- FR-A- 1 215 320
- GB-A- 925 889
- US-A- 3 730 181

## Description

The invention relates to a hearing protector as defined in the first part of the claim.

A hearing protector of this type is disclosed in FR-A-1.295.320. The meandrous air passage through the acoustic filter in this hearing protector is extending in one single (radial) direction. As a consequence of this the sound damping effect is not very large.

The invention aims at providing a hearing protector of the type above referred to, which better complies with each of the two, fundamentally contradictory requirements with to ventilation and sound damping.

More particularly the invention aims at providing a hearing protector, in which the meandrous air path in the sound damping acoustic filter does not suffer from the objections mentioned hereinabove.

The hearing protector according to the invention is characterized by the features in the second part of the claim.

The meandrous flow path obtained therewith has been found to work effectively in a diffusing and damping manner on the entering wave trains also in case of a relatively large ventilation opening.

An advantage of the hearing protector according to the invention is also to be seen in that the air path through the filter has a large cross-sectional area, so that sound pressure differences between the environment and the interior of the auditory canal will be smoothed out quicker. Moreover this construction with interengaging rings offer the possibility to adjust the penetration depth of the rings, so as to regulate the damping of the filter.

The invention will be hereinafter further explained by way of example with reference to the accompanying drawing.

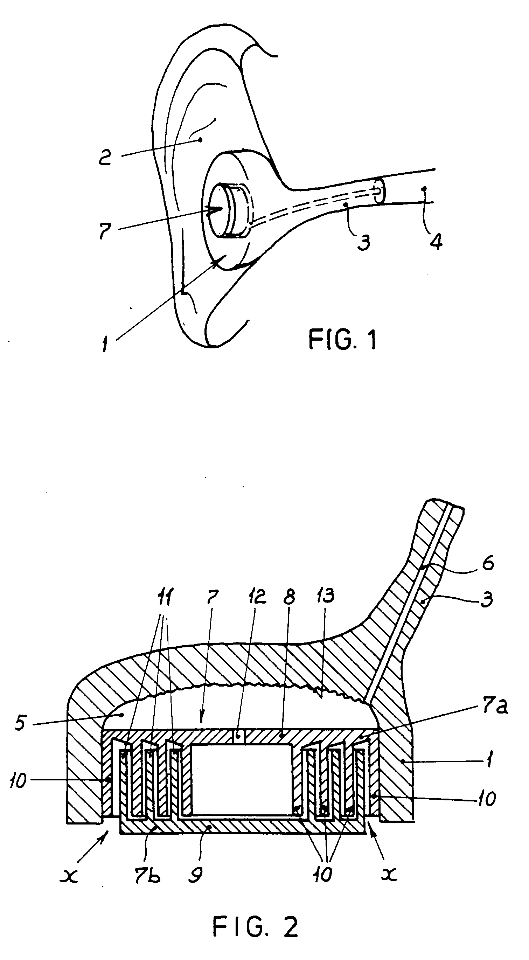

Fig. 1 is a perspective view of the hearing protector according to the invention and
Fig. 2 shows a cross-section on an enlarged scale and according to the axis of the acoustic filter of the hearing protector of Fig. 1.

The hearing protector shown in the drawing comprises a housing portion 1, adapted to be applied to the exterior of the auricle 2, and a portion 3 projecting from said first portion, which fits in the auditory canal 4. The portions 1 and 3 constitute an article which may be formed, according to well-known techniques - as a "made-to-measure" molded piece of plastics material, such as an acrylate resin.

The housing portion 1 has a chamber 5 which is formed e.g. by a substantially cylindrical recess. The projecting portion 3 has a passage 6 obtained e.g. by drilling, which starts in the chamber and merges at the free end of said portion 3. The passage 6 provides a free air connection between the chamber 5 on one hand and the portion of the auditory canal 4 behind the portion 3 on the other hand.

With the exception of the air connection to be described hereinafter, the chamber 5 is closed, towards the outside, by an acoustic filter 7. In the specific example of the invention this acoustic filter is composed of two parts 7a and 7b, each comprising a disc 8 and 9 respectively having axially projecting concentric rings 10 and 11 respectively. The disc 8 of one of said parts 7a, 7b is provided with a central opening 12 of a relatively wide cross-section. The two parts 7a and 7b engage into each other in a comb-like manner, whereby each ring 11 of the part 7b projects into an annular space between two adjacent rings 10 of the part 7a. In the example shown in the drawing the outer ring or wall 10 of the filter part 7a fits in an air-tight manner to the inner circumferential wall of the chamber 5.

Thus an acoustic filter is obtained, which comprises a plurality (in the illustrative example 7) of series-connected resonance cavities, which are capable of providing an effective sound damping and consequently an effective protection of the hearing against excessive sound. The two filter parts 7a and 7b should be designed so that the rings 10 and 11 will engage into one another with a slight clearance.

Furthermore the dimensions, in particular the heights of the rings 10 and 11, have to be selected so, that the multiples of quarter wave lengths of the frequences to be damped are avoided.

Air carrying sound energy which is to be kept out of the auditory canal as much as possible, may enter into the filter at x along the entire circumference and may from there flow, as through a labyrint, in radial direction towards the central air passage opening 12. In spite of the slight clearance between the interengaging rings 9 and 10, required for an effective sound damping, the total air passage opening, measured along the entire circumference of the filter, is of such an extent, that a practically free air connection with the auditory canal is secured.

As shown in the drawing, the bottoms of the grooves between the rings 10 of the inner filter part 7a are placed at an angle (of about 20°) the effect of this is, that incoming waves are reflected in a direction opposite to the entering air. The bottoms of the annular spaces between the rings 11 may have a correspondingly sloping position.

The bottom 13 of the chamber 5 is provided with an elastic coating having a rough surface. This produces an additional damping effect on the sound energy carried by the air passing through the acoustic filter.

It will be clear, that the filter - different from the situation shown in the drawings - may also be mounted with the central opening 12 directed to the outside. In that event air will enter at the center of the filter and will thereafter flow radially outwardly along the entire circumference into the chamber 5. It will be understood, that the slope of the bottoms of the grooves between the rings 10 has to be reversed in that event. This manner of mounting has the practical advantage, that the outer filter part 7a, may be simply fastened in the housing portion 1 with its outer ring or wall 10, e.g. by gluewing, whereas the inner filter part will then be kept in place automatically.

Just like the well-known filters, the filter according to the present invention may be easily replaced by another filter, the damping properties being adapted to the specific situation.

## Claims

1. A hearing protector comprising a housing having an outer portion (1) adapted to be applied against the auricle (2) and an inner portion (3) adapted to fit in the auditory canal (4) and has a passage (6) that emanates at its free inner end, said outer portion (1) having an outwardly opening chamber (5) in which a two-part (7a, 7b) sound damping acoustic filter (7) is provided, a substantially radially extending meandrous air passage through the acoustic filter (7) being formed as a labyrinth between the circumference at one extremity (x) of the filter body and a central opening (12) at the other extremity of the filter opening, said central opening (12) being connected with said passage (6) in said inner portion (3) so as to form an air connection between the auricle and the inner auditory canal, characterized in that the two parts (7a, 7b) of the acoustic filter (7) each comprises a disc member (8; 9) having axially extending concentric rings (10; 11), wherein the rings of one disc member project into the spaces between the rings of the other disc in a comb-like manner, the outer ring (10) of one (8) of the disc members being in air-tight sealing contact with the inner circumferential wall of the chamber (5), whereas the respective disc member (8) comprises said central opening (12).

## Patentansprüche

1. Gehörschützer, versehen mit einem Gehäuse mit einem zum Anlegen gegen die Ohrmuschel (2) geeigneten äusseren Teil (1) and einem in den Gehörgang (4) passenden inneren Teil (3), der einen am freien Ende desselben mündenden Kanal (6) aufweist, wobei der äussere Teil (1) eine nach aussen offene Kammer (5) aufweist, in der ein aus zwei Teilen (7a, 7b) zusammengestzter Schallfilter (7) vorgesehen ist, wobei ein sich etwa radial erstreckender mäandrischer Luftdurchgang wie ein Labirinth zwischen dem Umfang an einem Ende (x) des Filterkorpers und einer Zentralöffnung (12) am anderen Ende der Filteröffnung gebildet ist, welche Zentralöffnung (12) mit dem Kanal (6) im inneren Teil (3) verbunden ist zur Bildung einer Luftverbindung zwischen der Ohrmuschel und dem inneren Gehörgang, dadurch gekennzeichnet, dass die beiden Teile (7a, 7b) des Schallfilters (7) je ein scheibenförmiges Element (8; 9) mit in achsialer Richtung vom diesem ausragenden konzentrischen Ringen (10; 11) aufweisen, wobei die Ringe von einem scheibenförmigen Element kammartig in den Räumen zwischen den Ringen des anderen Scheibenelementes stecken, und wobei der äussere Ring (10) eines der beiden Scheibenelements luftdicht gegen die innere Umfangswand der Kammer (5) abdichtet und das betreffende Scheibenelement (8) die ganannte Zentralöffnung (12) aufweist.

## Revendications

1. Un protecteur auditif comprenant un boîtier ayant une partie extérieure (1) conçue pour être appliquée contre le pavillon de l'oreille (2), et une partie intérieure (3) qui est conçue pour s'adapter dans le conduit auditif (4) et qui comporte un passage (6) qui débouche à son extremité intérieure libre, la partie extérieure (1) comportant une chambre (5) s'ouvrant vers l'extérieur, dans laquelle est incorporé un filtre acoustique d'amortissement du son (7) en des parties (7a, 7b), un passage d'air en méandres d'étendent en direction pratiquement radiale à travers le filtre acoustique (7) étant formé à la manière d'un labyrinthe entre la circonférence à une extrémité (x) du corps du filtre, et une ouverture centrale (12) à l'autre extrémité du corps du filtre, cette ouverture centrale (12) étant reliée au passage précité (6) dans la partie intérieure (3), de façon à former un passage d'air entre le pavillon de l'oreille et le conduit auditif interne, caractérisé en ce que chacune des deux parties (7a, 7b) du filtre acoustique (7) comprend un élément en forme de disque (8; 9) portant des anneaux concentriques (10; 11) qui s'étendent en direction axiale, les anneaux d'un élément en forme de disque s'étendant à l'intérieur des espaces entre les anneaux de l'autre élément en forme de disque, en une configuration en peigne, et l'anneau extérieur (10) de l'un (8) des éléments en forme de disque étant en contact hermétique avec la paroi de la circonférence intérieure de la chambre (5), tandis que l'élément en forme de disque respectif (8) comprend l'ouverture centrale (12).
